# EUROPEAN PATENT SPECIFICATION

(11) **EP 4 489 659 B1**
(45) Date of publication and mention of the grant of the patent: **19.08.2026**
(21) Application number: 23715387.9
(22) Date of filing: 10.03.2023
(51) Int. Cl.: A61B 8/04, A61B 7/00, A61B 5/021, A61B 5/00, A61B 8/00, A61B 8/08

(54) **CONTINUOUS NONINVASIVE BLOOD PRESSURE MEASUREMENT**
KONTINUIERLICHE NICHTINVASIVE BLUTDRUCKMESSUNG
MESURE CONTINUE NON INVASIVE DE LA PRESSION ARTÉRIELLE

(30) Priority: 11.03.2022 US 202263319259 P
(43) Date of publication of application: 15.01.2025
(73) Proprietor: Masimo Corporation, Irvine, CA 92618 (US)
(72) Inventor: TELFORT, Valery G., Irvine, California 92618 (US)
(74) Representative: Finlayson, Scott Henry
(86) International application number: PCT/US2023/064198
(87) International publication number: WO 2023/173128

(56) References cited:
- US-B1- 6 275 447

## Description

### BACKGROUND

### Field

This disclosure relates to a blood pressure monitoring system.

### Description of the Related Art

The human cardiovascular system is made up of the heart, blood vessels, and blood. The heart pumps blood through the blood vessels in order to transport oxygen, nutrients, etc., throughout the body.

Blood pressure is a measure of the pressure exerted by the circulating blood on the walls of the blood vessels and is typically measured in one of the large arteries. Blood pressure varies during the cardiac cycle from one heartbeat to the next. When the heart contracts, blood pressure momentarily rises and then subsequently falls until the next heartbeat. The systolic pressure is the maximum blood pressure attained during a cardiac cycle, while the diastolic pressure is the minimum blood pressure during the cardiac cycle. The mean arterial pressure (MAP) is the average blood pressure during the cardiac cycle. Blood pressure depends on a number of factors, including blood volume, cardiac output, vascular resistance, arterial stiffness, etc.

In medicine, blood pressure is a vital sign which can be used as an indicator of a patient's condition. Improved devices and techniques for measuring blood pressure can therefore help improve patient monitoring capabilities.

US6275447 describes, in accordance with its abstract, a sound generating apparatus or a sound detecting apparatus which includes: a flexible substrate; and a plurality of bimorphs arranged on a surface of the flexible substrate in a direction at a predetermined interval, each of the bimorphs having input/output terminals. Each of the bimorphs has a rectangular shape and its longitudinal direction is perpendicular to the direction. The flexible substrate has a slit between successive two of the bimorphs. A width of the slit outside the bimorphs is wide. A buffering material covering surfaces of the flexible substrate with the bimorphs exposed or a cover covering the bimorphs with a space may be provided. The cover and the buffering material may have slit. A sound generating apparatus including a plurality of flexible substrates respectively supporting bimorphs may be provided. The flexible substrate may have a comb shape. An acoustic sensor includes the sound generating apparatus, the sound detection apparatus connected therebetween by a flexible substrate, wherein the flexible substrate may include a cable and a connector. A living body measuring apparatus includes the acoustic sensor, wherein phase shift between the sound signal induced by the bimorph and a reference drive signal is detected and a blood pressure is operated by the phase shift amount.

### SUMMARY

The invention is defined in the appended claims.

### BRIEF DESCRIPTION OF THE DRAWINGS

FIG. 1A is a schematic illustration of the anatomy of a human arm.
FIG. 1B is a cross-sectional view of the human arm shown in FIG. 1A near the wrist.
FIG. 1C shows an embodiment of a blood pressure monitoring system, which includes an acoustic exciter and an acoustic detector positioned spaced apart from one another but both adjacent to the radial artery in a patient's arm.
FIG. 1D schematically illustrates propagation of the acoustic signal from the acoustic exciter through the patient's body to the acoustic detector.
FIG. 1E illustrates an example embodiment of a sensor for the blood pressure monitoring system.
FIG. 1F illustrates another example embodiment of a sensor for the blood pressure monitoring system.
FIG. 1G illustrates exploded and assembled views of another example embodiment of a sensor for the blood pressure monitoring system.
FIG. 1H shows plan and edge views of the sensor that is illustrated in FIG. 1G.
FIG. 1I illustrates the sensor illustrated in FIG. 1G in the as-worn position on a patient's forearm.
FIG. 2 shows graphs of an example electrical input signal to the acoustic exciter (top graph), the resulting acoustic input signal which is outputted from the acoustic exciter (middle graph), and an acoustic output signal from an acoustic detector (bottom graph).
FIGS. 3A-3D illustrate how the array of acoustic detectors (e.g., as shown in FIGS. 1F and 1G) can be used to improve measurements of the blood pressure monitoring system 100 described herein.
FIG. 4A illustrates acoustic emitter-detector separation distances for an embodiment of the sensor illustrated in FIG. 1G.
FIG. 4B is a graph of the elapsed phase detected by the sensor in FIG. 4A for each of ten frequency components at each of the four acoustic detectors for a particular moment in time.
FIG. 4C is a graph which further illustrates how the elapsed phase values measured by the acoustic detectors of the sensor in FIG. 4A for each frequency component can be used to determine slope values for lines of best fit so as to determine blood pressure values.
FIG. 4D is another graph which further illustrates how the elapsed phase values measured by the acoustic detectors of the sensor in FIG. 4A for each frequency component can be used to determine slope values for lines of best fit so as to determine blood pressure values.
FIG. 4E is a graph which illustrates how the elapsed phase values measured by the acoustic detectors of the sensor in FIG. 4A for each frequency component can be used to determine slope values for planes of best fit so as to determine blood pressure values.
FIGS. 5A-5D illustrate example embodiments of sensors for the blood pressure monitoring system.
FIGS. 6A-6B illustrate an example embodiment of a blood pressure monitoring system with a built-in display.
FIGS. 7A-7C illustrate an example embodiment of a blood pressure monitoring system with an acoustic exciter and acoustic detectors integrated in a wristband.
FIGS. 8A-8C illustrate an example embodiment of a blood pressure monitoring system which is similar to that of FIGS. 7A-7C but which additionally includes a built-in display.

### DETAILED DESCRIPTION

Various embodiments of systems and methods for continuously and noninvasively measuring a patient's blood pressure are described herein.

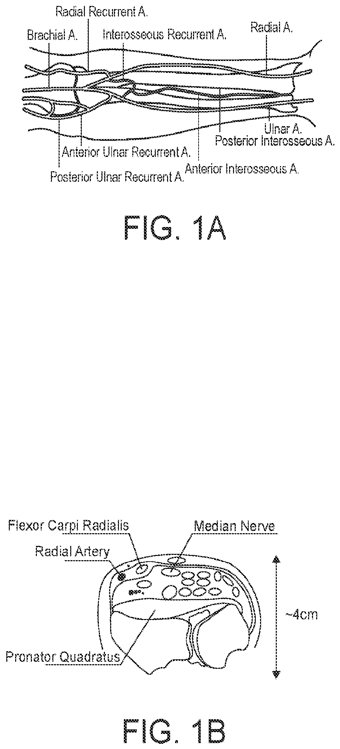

FIG. 1A is a schematic illustration of the anatomy of a human arm. The radius and ulna bones are shown. Several arteries are also illustrated, including the radial artery. The radial artery is a blood vessel that supplies oxygenated, pulsing blood to the forearm and hand. In some embodiments, the blood pressure monitoring systems described herein can be used to measure a patient's blood pressure in the radial artery (e.g., at the lower forearm). The monitoring systems are also compatible with other arteries (e.g., the brachial artery at the upper arm) and monitoring sites, however.

FIG. 1B is a cross-sectional view of the human arm shown in FIG. 1A near the wrist. Various anatomical features are shown, including the radius and ulna bones, the radial artery, the flexor carpi radialis muscle, the pronator quadratus muscle, and the median nerve. A typical cross-sectional thickness of a human arm near the wrist is ~4 cm. As shown in FIG. 1B, the portion of the radial artery near the wrist is located over the radius bone and is relatively close to the surface of the skin. The location of the radial artery makes it a relatively good monitoring site for measuring a patient's blood pressure.

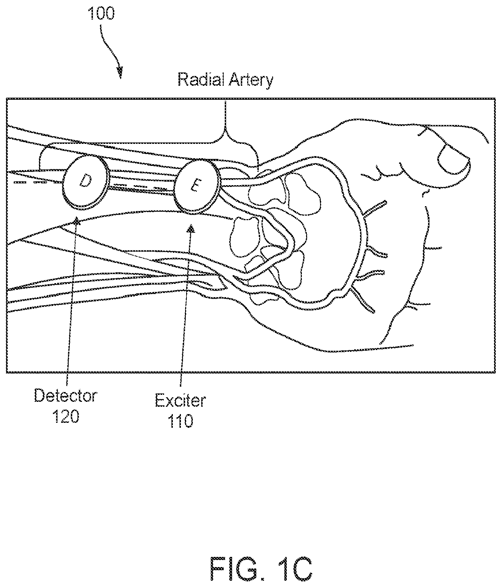

FIG. 1C shows an embodiment of a blood pressure monitoring system 100, which includes an acoustic exciter 110 and an acoustic detector 120 positioned spaced apart from one another but both adjacent to the radial artery in a patient's arm. Although not illustrated, the blood pressure monitoring system 100 can also include other elements, such as a signal/waveform generator connected to the acoustic exciter 110 so as to provide a desired input signal, as well as a processor connected to the acoustic detector 120 to analyze the detected output signal. In addition, the blood pressure monitoring system 100 can include a power source, such as a battery, a digital-to-analog converter to convert a digital signal from the signal/waveform generator to an analog electrical signal, an analog-to-digital converter to convert an electrical signal from the acoustic detector 120 to a digital signal, a display, signal leads, an attachment tape, strap, or other element for attaching the system to the patient, etc. In some embodiments, the blood pressure monitoring system 100 also includes an accelerometer and/or gyroscope. These devices can be used to determine whether the patient is moving, which may result in an inaccurate measurement. For example, if the accelerometer and/or gyroscope detect a degree of motion above a selected threshold, blood pressure data or measurements collected during the motion may be disregarded or deemphasized by the system. The accelerometer and/or gyroscope can also be used to estimate the position of the patient's arm, which can in turn be used to correct blood pressure measurements for differences in hydrostatic pressure depending on the patient's arm pose.

The blood pressure monitoring system 100 can be used to noninvasively monitor the patient's blood pressure in the radial artery, as described further herein. The blood pressure monitoring system 100 can provide a relatively continuous (e.g., real-time) measurement waveform of the patient's blood pressure. In some embodiments, the blood pressure monitoring system 100 provides measurements of the instantaneous blood pressure, the systolic blood pressure, the diastolic blood pressure, the mean arterial pressure, and/or any other blood pressure metric. In some embodiments, the measurement output of the blood pressure monitoring system 100 can be compared with that of an invasive direct arterial line, and can be contrasted with the more intermittent measurements provided by a noninvasive cuff-based measurement device.

As shown in FIG. 1C, the acoustic exciter 110 and the acoustic detector 120 are located on the inside of the patient's forearm, typically as close as possible/practicable to the radial artery. For example, the acoustic exciter 110 and the acoustic detector 120 can be placed directly over the radial artery. The acoustic exciter 110 and the acoustic detector 120 are separated from one another along the length of the radial artery by a separation distance, d, which can be, for example, 1-15 cm, though other separation distances are also possible. Both the acoustic exciter 110 and the acoustic detector 120 can be acoustically and/or mechanically coupled with the patient's body. The acoustic exciter 110 can be a transducer which converts electrical energy from a power source and an electrical signal from an electrical signal/waveform generator into acoustic and/or mechanical energy as an input acoustic signal. In some embodiments, the acoustic exciter 110 is a piezo device or a microelectromechanical system (MEMS). Other types of acoustic exciters can also be used. The acoustic exciter 110 emits the input acoustic signal, which is coupled into the body and then propagates via multiple paths to the acoustic detector 120. The acoustic detector 120 can be a transducer which converts acoustic and/or mechanical energy into electrical energy as an electrical output signal. In some embodiments, the acoustic detector 120 is also a piezo device or a microelectromechanical system (MEMS), though other types of acoustic detectors can be used. The electrical output signal from the acoustic detector 120 can be provided to a processor (e.g., after being digitized) for analysis by one or more algorithms.

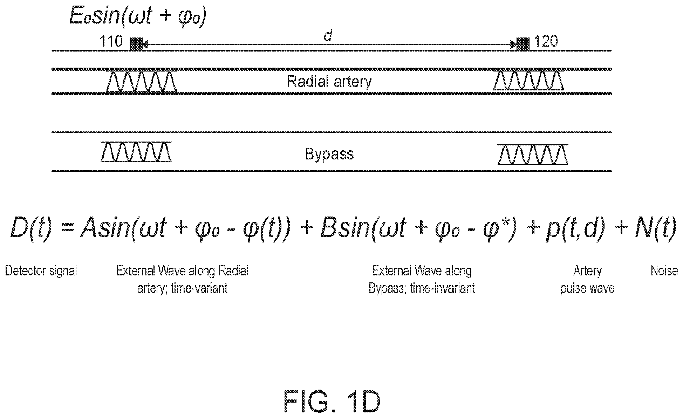

FIG. 1D schematically illustrates propagation of the acoustic signal from the acoustic exciter 110 through the patient's body to the acoustic detector 120. As shown in FIG. 1D, the acoustic exciter 110 and the acoustic detector 120 can be placed in contact with the patient's skin over the radial artery. A distance d separates the acoustic exciter 110 and the acoustic detector 120. The acoustic signal from the acoustic exciter 110 can propagate to the acoustic detector 120 via multiple different paths. For example, as shown in FIG. 1D, a component of the acoustic signal can propagate to the acoustic detector 120 primarily via the radial artery. Other components of the acoustic signal can propagate to the acoustic detector 120 via bone, skin, muscle, and other tissues in the body. These paths are collectively illustrated as the bypass path shown in FIG. 1D. Though not illustrated, still other components of the acoustic signal may propagate to the acoustic detector 120 via external structures, such as sensor substrates, adhesive tape, etc. which are part of the blood pressure measurement system 100 itself. Such signal paths may also be characterized as bypass paths.

The component of the output signal which propagates from the acoustic exciter 110 to the acoustic detector 120 primarily via the radial artery is affected by the instantaneous blood pressure within the radial artery. This is because the instantaneous blood pressure in the artery affects the stiffness of the arterial walls, which in turn affects the wave speed of the acoustic signal which propagates via the artery. Since the instantaneous blood pressure in the radial artery pulses with the patient's heartbeat, the acoustic signal which propagates via the radial artery is modulated by the patient's pulse. The component of the acoustic signal which propagates via the radial artery is represented mathematically by the first term of the detector signal, D(t), as shown in FIG. 1D. This term includes a time-varying phase term which represents the modulation of the input signal by the patient's pulse. The components of the acoustic signal which propagate to the acoustic detector 120 via any of several bypass paths are represented mathematically by the second term of the detector signal, D(t), as shown in FIG. 1D. The bypass paths are expected to be relatively constant, so the bypass term does not include a time-varying phase term.

The modulation introduced by the pulsing of the radial artery allows the component of the output signal which propagated to the acoustic detector 120 via the radial artery to be separated from the other components of the output signal which propagated to the acoustic detector via other paths. In some embodiments, the processing algorithm(s) implemented by the processor which receives the output of the acoustic detector 120 are used to isolate the component of the output signal which propagated primarily via the radial artery from the other components. In some embodiments, the modulation introduced by the pulsing of the radial artery can be understood as phase modulation which introduces a phase and/or time delay in the acoustic signal as it propagates from the acoustic exciter 110 to the acoustic detector 120.

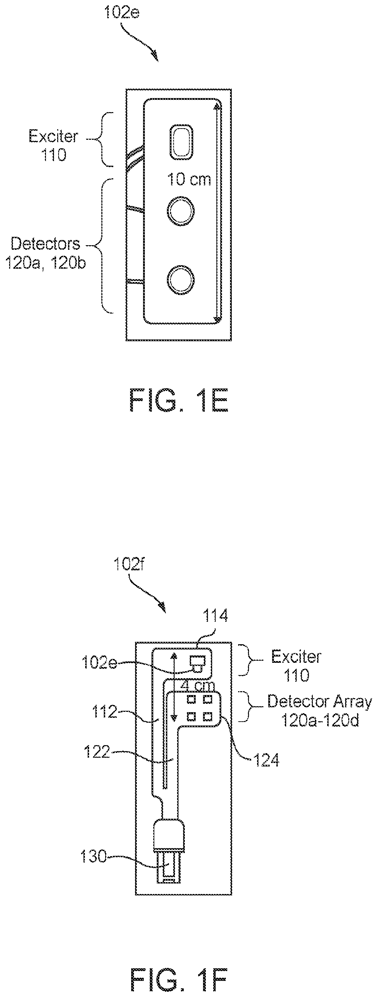

FIG. 1E illustrates an example embodiment of a sensor 102e for the blood pressure monitoring system 100. Sensor 102e includes an acoustic exciter 110 and two acoustic detectors 120a, 120b, all provided on a common sensor substrate. Signal leads extend from the sensor substrate and connect the sensor 102e to a signal/waveform generator, processor, power supply, etc. (not shown). The acoustic exciter 110 and the two acoustic detectors 120a, 120b are arranged on the common sensor substrate along a linear path. The length of the substrate is ~10 cm. Sensor 102e can be positioned on the inside of the patient's lower arm over the radial artery using, for example, tape or a strap.

FIG. 1F illustrates another example embodiment of a sensor 102f for the blood pressure monitoring system 100. Sensor 102f includes an acoustic exciter 110 and a detector array which consists of a two-by-two grid of four acoustic detectors 120a-120d. Although a two-by-two grid of acoustic detectors 120 is illustrated, more or fewer acoustic detectors can also be used and the detectors may be arranged in different arrays, including linear arrays. Sensor 102f also includes an acoustic detector 120e located directly adjacent (e.g., within 5 mm, or within 2 mm, or within 1 mm) to the acoustic exciter 110. The respective purposes of the acoustic detector array 120a-120d and the acoustic detector 120e adjacent to the acoustic exciter 110 are discussed further below. In the illustrated embodiment, the acoustic exciter 110 and its adjacent acoustic detector 120e are aligned with the center point of the detector array 120, though other layouts are also possible.

The components of sensor 102f are provided on a substrate. For example, the acoustic exciter 110 is provided on an exciter portion 114 of the substrate, while the detector array 120 is provided on a detector portion 124 of the substrate. Sensor 102f also includes a first bypass portion 112 of the substrate and a second bypass portion 122 of the substrate. In some embodiments, the first and second bypass portions 112, 122 of the substrate can be elongate arm or lead type structures. The first and second bypass portions 112, 122 are connected to the exciter portion 114 and the detector portion 124 at their respective distal ends and are connected to one another at their proximal ends, near the sensor's electrical connector. As discussed further below, the first and second bypass portions 112, 122 can be used to provide structural support for the exciter portion 114 and the detector portion 124 of the substrate, but to do so in a manner that provides a bypass acoustic signal path from the acoustic exciter 110 to the acoustic detector(s) 120a-d that is longer (e.g., substantially longer) than the straight-line distance between the acoustic exciter 110 and the acoustic detector(s) 120a-d.

The acoustic exciter 110 and the acoustic detectors 120 in sensor 102f are physically smaller devices than the corresponding elements in sensor 102e shown in FIG. 1E. As a result of using smaller devices, sensor 102f in FIG. 1F is better suited to the use of acoustic signals with higher frequencies. This in turn allows for a smaller distance, d, between the acoustic exciter 110 and the acoustic detector array 120. As shown in FIG. 1F, the separation distance, d, in this embodiment is less than 4 cm. Specifically, in the illustrated embodiment, the distance, d, between the acoustic exciter 110 and the center of the array of acoustic detectors 120a-120d is 2.5 cm. Other separation distances can also be used. Longer separation distances lengthen the measured propagation delay of the acoustic signal, which can improve signal-to-noise ratio. However, longer separation distances can also increase attenuation of the acoustic signal, which in turn can worsen the signal-to-noise ratio. This tradeoff can be balanced according to each application of the blood pressure monitoring system 100. The separation distance can also be dependent upon the acoustic signal frequency. For example, higher frequencies can allow for the use of smaller separation distances.

As discussed above, the acoustic exciter 110 and the acoustic detectors 120 of sensor 102f are mounted on substrate portions 114, 124 which are in turn connected to bypass portions (e.g., arms or leads) 112, 122 of the substrate. These substrate portions include signal traces for conducting electrical input signals to the acoustic exciter 110 and for conducting electrical output signals from the acoustic detectors 120. In some embodiments, the substrate portions and signal traces are flexible to allow sensor 102f to conform to the patient's anatomy at the monitoring site.

In the illustrated embodiment, the first bypass portion 112 of the substrate (connecting to the acoustic exciter 110) is physically split from the second bypass portion 122 of the substrate (connecting to the acoustic detector(s) 120). In the illustrated embodiment, there is a gap (e.g., in the direct direction from the acoustic exciter 110 to the acoustic detector array 120) between the two bypass substrate portions 112, 122 such that they are mechanically and/or acoustically decoupled. This helps acoustically isolate the acoustic detector(s) 120 from the acoustic exciter 110. In the FIG. 1F embodiment, the gap between the first and second bypass substrate portions 112, 122 extends toward the plug side of sensor 102f by a distance that is a multiple of the straight-line distance between the acoustic exciter 110 and the acoustic detector array 120. The split arrangement of the bypass arms/leads helps attenuate bypass acoustic signals which may otherwise propagate from the acoustic exciter 110 to the acoustic detectors 120 via vibrations in the substrate portions of sensor 102f. For example, the split bypass arm/lead arrangement can be designed such that the path from the acoustic exciter 110 to the acoustic detectors 120 via the patient's body (e.g., in a straight line from exciter to detector) is significantly shorter than the loopback path from the acoustic exciter 110 to the acoustic detectors 120 via the substrate portions 112, 122. Due to the much greater path length, acoustic bypass signals which propagate from the acoustic exciter 110 to the acoustic detectors 120 via the bypass substrate portions 112, 122 can be attenuated to a greater degree than the desired signal which travels to the acoustic detectors 120 via the radial artery. This can improve performance of the blood pressure monitoring system 100.

In some embodiments, sensor 102f can be designed such that the bypass path from the acoustic exciter 110 to the acoustic detectors 120 via the substrate portions 112, 122 is greater than 2x, greater than 5x, or greater than 10x the physiological path distance (e.g., the straight-line distance) between the acoustic exciter 110 and the acoustic detectors 120. To further improve performance, the substrate portions 112, 122 can include one or more acoustic materials and/or dampening masses designed to absorb vibrations and further acoustically isolate the acoustic exciter 110 from the acoustic detectors 120. Acoustic absorbing material and/or vibration dampening mass can be provided, for example, at any location along the loopback path from the acoustic exciter 110 to the acoustic detector(s) 120 via any structure of the sensor.

While FIG. 1F shows a physical gap between the first bypass substrate portion 112 for the acoustic exciter 110 and the second bypass substrate portion 122 for the acoustic detectors 120, in other embodiments it may be that a sensor for the blood pressure monitoring system 100 is designed with a shared substrate portion for both the acoustic exciter 110 and the acoustic detectors 120 (e.g., as in FIG. 1E). For example, in such embodiments, there may instead be an acoustic barrier which can be achieved by providing an acoustic absorber material and/or dampening mass between the acoustic exciter 110 and the acoustic detectors 120. In still other embodiments, a combination of open space (e.g., gaps), acoustical absorbing materials, and/or dampening masses can be used to provide mechanical de-coupling and/or acoustic isolation between the acoustic exciter 110 and the acoustic detectors 120.

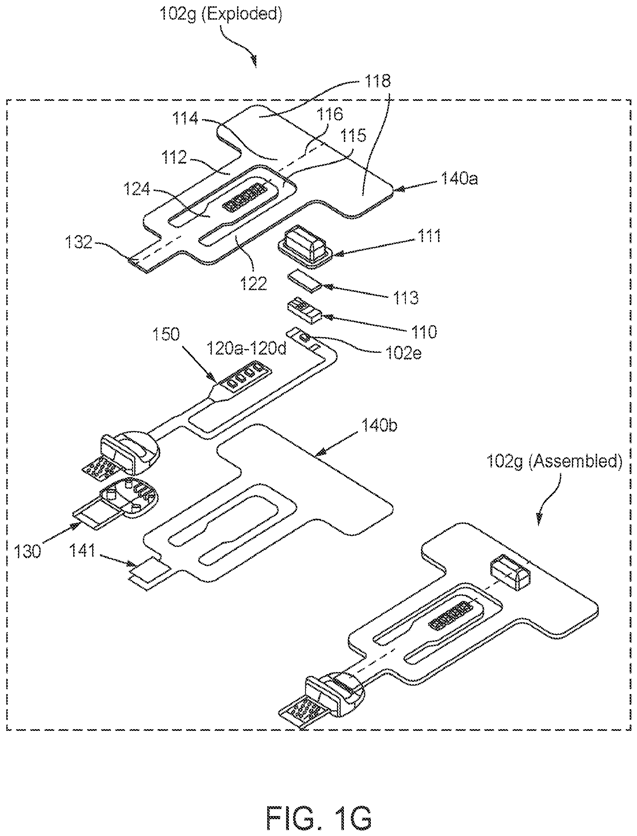

FIG. 1G illustrates exploded and assembled views of another example embodiment of a sensor 102g for the blood pressure monitoring system 100. Sensor 102g includes an acoustic exciter 110 and a linear detector array which consists of four acoustic detectors 120a-120d. The acoustic exciter 110 and the acoustic detectors 120a-e can be mounted to a flexible circuit layer 150. A protective cover 111 can be provided over the acoustic exciter 110. A layer of foam 113 can be provided between the acoustic exciter 110 and the protective cover 111 to provide acoustic isolation and prevent resonant vibration of the protective cover 111. Sensor 102g also includes an acoustic detector 120e located underneath the acoustic exciter 110. The respective purposes of the acoustic detector array 120a-120d and the acoustic detector 120e adjacent to the acoustic exciter 110 are discussed further below. In the illustrated embodiment, the acoustic exciter 110 and its adjacent acoustic detector 120e are aligned with the axis of the linear detector array 120, though other layouts are also possible.

The components of sensor 102g are provided on a substrate. As illustrated in the exploded view of FIG. 1G, the substrate can include multiple layers, including the flexible circuit layer 150. The top and bottom layers 140a, 140b of the substrate may be formed of a flexible material, such as relatively thin layers of foam, silicone, plastic, etc. The bottom layer 140b may include adhesive on its bottom surface so as to enable sensor 102g to be attached to a patient's arm. The adhesive may be covered by a peel off cover which can be removed by a clinician when placing the sensor. The bottom layer 140b can also include windows directly underneath the acoustic exciter 110 and the acoustic detectors 120a-e so as to allow them better access to the patient's skin, though this is not necessarily required. A flexible circuit 150 can be provided between the top and bottom layers 140a, 140b of the substrate. The flexible circuit can include electrical traces to electrically connect the acoustic exciter 110 and the acoustic detectors 120a-e to the connector 130, which can in turn connect to the battery, processor, signal/waveform generator, etc. A foam pad can be provided under the connector 130 for strain relief.

The acoustic exciter 110 is provided on an exciter portion 114 of the substrate, while the detector array 120a-d is provided on a detector portion 124 of the substrate. Sensor 102g also includes a first bypass portion 112 of the substrate and a second bypass portion 122 of the substrate. In some embodiments, the first and second bypass portions 112, 122 of the substrate can be elongate arm or lead type structures. The first and second bypass portions 112, 122 are both connected to the exciter portion 114 at their respective distal ends and are also both connected to one another and to the detector portion 124 of the substrate at their proximal ends, near the connector portion 132 of the substrate, which in turn mechanically couples with the sensor's electrical connector 130. This arrangement where the distal ends of both bypass portions 112, 122 of the substrate are connected to the exciter portion 114 of the substrate, and both proximal ends are connected to the detector portion 124 of the substrate, can be beneficial because the acoustic exciter 110 can be more firmly held in a fixed spatial relationship with respect to the acoustic detector array 120a-d, as opposed to the arrangement shown in FIG. 1F where any relative transverse bending or displacement between the bypass portions 112, 122 could result in misalignment between the acoustic exciter 110 and the acoustic detector array 120a-d. As already discussed, the first and second bypass portions 112, 122 can be used to provide structural support for the exciter portion 114 and the detector portion 124 of the substrate, but do so in a manner that provides a bypass acoustic signal path from the acoustic exciter 110 to the acoustic detector(s) 120a-d that is longer (e.g., substantially longer) than the straight-line distance between the acoustic exciter 110 and the acoustic detector(s) 120a-d. As illustrated in FIG. 1G, the flexible circuit 150 can also include a bypass portion underneath bypass portion 122.

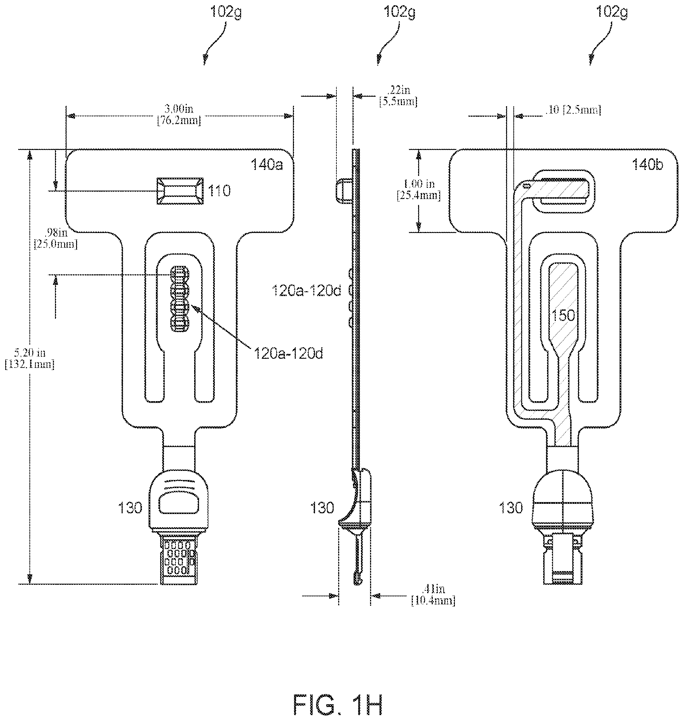

FIG. 1H shows plan and edge views of the sensor 102g that is illustrated in FIG. 1G. FIG. 1H includes example dimensions of various features. It should be understood, however, that other dimensions can also be used.

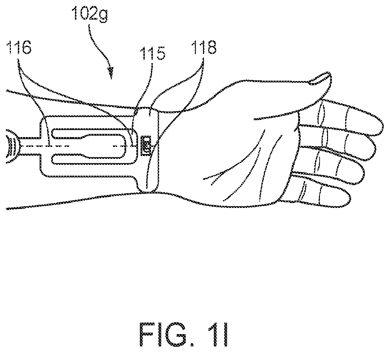

FIG. 1I illustrates the sensor 102g (illustrated in FIG. 1G) in the as-wom position on a patient's forearm. In the illustrated embodiment, sensor 102g is attached to the arm with adhesive provided on the bottom surface of the sensor. The substrate of sensor 102g can include wing portions which extend laterally away from the measurement axis of the sensor and wrap at least partially around the patient's arm to help fix the sensor securely in place.

Sensor 102g also includes an alignment window 115 and alignment indicator/indicia 116. In addition to helping to provide acoustic decoupling between the acoustic exciter 110 and the acoustic detector array 120a-d via a loopback path through the sensor substrate, the alignment window 118 can also be used by a clinician to align the measurement axis of sensor 102g (i.e., the axis from the acoustic exciter 110 to the linear array of acoustic detectors 120a-d) with the patient's radial artery. The alignment indicator/indicia 116 also can assist in the alignment process. In some embodiments, the clinician who is placing sensor 102g on the patient's arm can identify the location of the radial artery using, for example, palpation or ultrasound. In some cases, the clinician may mark the position of the radial artery with ink on the patient's arm. The clinician may then view the location of the radial artery through the alignment window 118 and can position sensor 102g such that the alignment indicator/indicia 116 lines up with the radial artery. In this way, the clinician can ensure that the measurement axis of sensor 102g is aligned with the radial artery. This can improve signal-to-noise ratio and measurement accuracy. Although FIG. 1I illustrates alignment of sensor 102g with the radial artery, the systems described herein can also be used with other arteries and measurement sites.

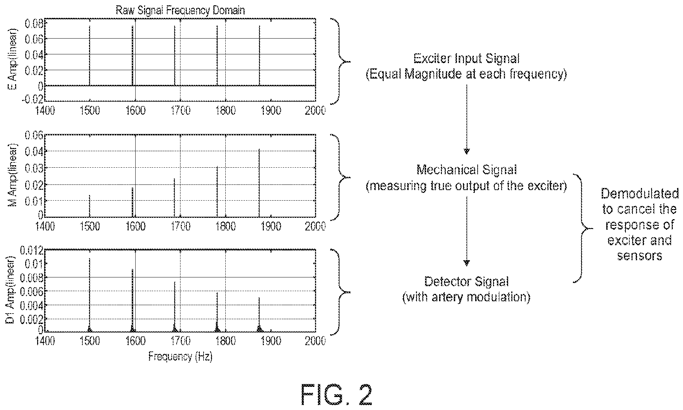

FIG. 2 shows graphs of an example electrical input signal to the acoustic exciter 110 (top graph), the resulting acoustic input signal which is outputted from the acoustic exciter 110 (middle graph), and an acoustic output signal from an acoustic detector 120 (bottom graph). Each of the graphs plots signal magnitude as a function of frequency. In the illustrated embodiment, the electrical input signal (top graph) consists of multiple equal power tones at regular frequency intervals. In other embodiments, more or fewer frequency tones could be used. More complex signals with broadband frequency content can also be used.

Although the top graph represents the electrical input signal applied to the acoustic exciter 110, the acoustic input signal (middle graph) produced by the acoustic exciter 110 will typically vary from the electrical input signal. In FIG. 2, this can be seen by comparison of the top graph with the middle graph. The difference between the electrical input signal and the acoustic input signal is due to the unique characteristics of each acoustic exciter 110. For example, the acoustic exciter 110 may produce harmonic distortion, which can lead to amplitude and/or phase differences between the electrical input signal (top graph) and the acoustic input signal (middle graph). The effect of the acoustic exciter 110 on the electrical input signal can be characterized by a transfer function which relates the electrical input signal to the acoustic input signal. If left unaccounted for, the difference between the electrical input signal and the acoustic input signal can introduce error in the blood pressure measurements captured by the system. Thus, sensor 102f in FIG. 1F includes detector 120e directly adjacent to the acoustic exciter 110 in order to measure the actual acoustic input signal produced by the acoustic exciter. In some embodiments, the processor uses the electrical output signal from detector 120e to determine one or more characteristics of the acoustic signal. The characteristic(s) of the acoustic signal can be used to adjust the electrical output signals from acoustic detectors 120a-d and/or the blood pressure measurement.

The bottom graph in FIG. 2 shows the electrical output signal captured by the acoustic detector 120a-d. Using the output of detector 120e, which represents the actual acoustic input signal, the detector signal can be demodulated so as to lessen or cancel the effect of the acoustic exciter response on the input electrical signal.

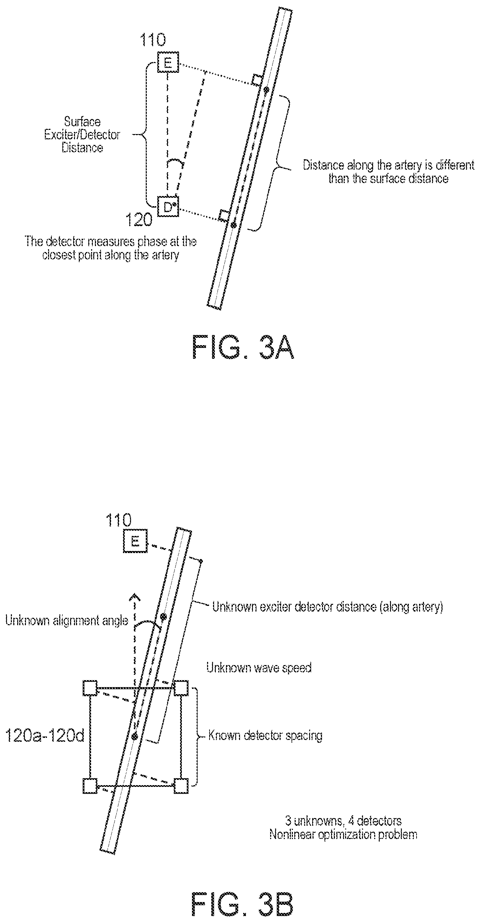

FIGS. 3A-3D illustrate how the array of acoustic detectors 120a-120d described herein (e.g., as shown in FIGS. 1F and 1G) can be used to improve measurements of the blood pressure monitoring system 100 described herein. FIG. 3A is a schematic diagram of the placement of the acoustic exciter 110 and an acoustic detector 120 with respect to the radial artery. As shown in the figure, the sensor (e.g., 102e, 102f) which carries the acoustic exciter 110 and the acoustic detector 120 may be misaligned with respect to the radial artery. Such misalignment is common because, for some patients, the clinician may not be able to tell the exact path of the radial artery just by visual inspection. The dashed line in FIG. 3A from the acoustic exciter 110 to the acoustic detector 120 represents the separation distance, d, between the acoustic exciter 110 and the acoustic detector 120. The separation distance, d, is known. The dashed projection line onto the artery in FIG. 3A represents the actual distance traveled by the acoustic signal via the radial artery. This distance is unknown, as is the misalignment angle of the sensor with respect to the radial artery.

The unknown propagation distance of the acoustic signal along the radial artery can introduce error into the blood pressure measurements captured by the blood pressure measurement system 100. This is because the blood pressure measurement system 100 measures the time for the acoustic signal to propagate from the acoustic exciter 110 to the acoustic detector 120 via the radial artery, yet the propagation speed is the quantity that is related to blood pressure in the artery. Thus, in order to calculate the propagation speed of the acoustic signal, the system divides the distance traveled by the propagation time. If there is misalignment between the sensor and the radial artery, use of the known separation distance, d, between the acoustic exciter 110 and the acoustic detector 120 in this calculation in place of the unknown actual propagation distance results in a measurement error.

FIG. 3B is a schematic diagram of the placement of the acoustic exciter 110 and the array of acoustic detectors 120a-120d with respect to the radial artery. As seen in the figure, there is a different distance between the acoustic exciter 110 and each of the acoustic detectors 120a-120d. In some embodiments, the output signal from the acoustic detector whose exciter-detector distance is closest to the actual unknown propagation distance of the acoustic signal along the radial artery can be used to perform the blood pressure measurement. The corresponding known exciter-detector distance of the selected acoustic detector can be used in the wave speed calculation. In such cases, one of the acoustic detectors 120a-120d can be selected based upon which provides the best output according to a selected metric.

In other embodiments, however, the outputs of all of the acoustic detectors 120a-120d can be used in order to estimate the unknown propagation distance of the acoustic signal along the radial artery, thereby canceling the effect of any misalignment between the sensor and the radial artery. As shown in FIG. 3B, the misalignment problem results in three unknown values (alignment angle, wave speed, and propagation distance along the artery) and four known detector output signals. By finding a solution to the resulting nonlinear optimization problem, the three unknown values can be estimated. And by using these estimates in the blood pressure measurement calculations, accuracy can be improved. This technique also reduces the dependency of blood pressure measurements on the placement of the sensor by the clinician, which improves ease of use as well.

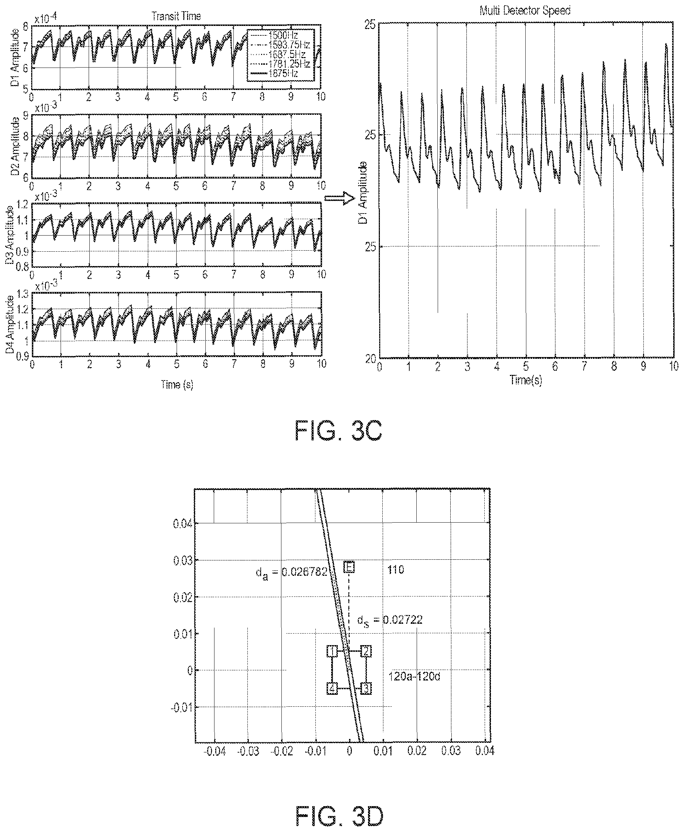

FIG. 3C illustrates how output signals from multiple acoustic detectors 120a-120d can be combined into a composite output signal with reduced dependence on sensor misalignment. Each of the four graphs on the left hand side of FIG. 3C is the output of one of the four acoustic detectors 120a-120d. By finding a solution to the nonlinear optimization problem represented by FIG. 3B, the four detector outputs are combined to form a composite output signal, as shown in the graph on the right hand side of FIG. 3C. The resulting composite output signal can be used in the blood pressure measurement calculations. FIG. 3D shows the estimated propagation distance of the acoustic signal along the radial artery, as calculated from the composite output signal shown in FIG. 3C.

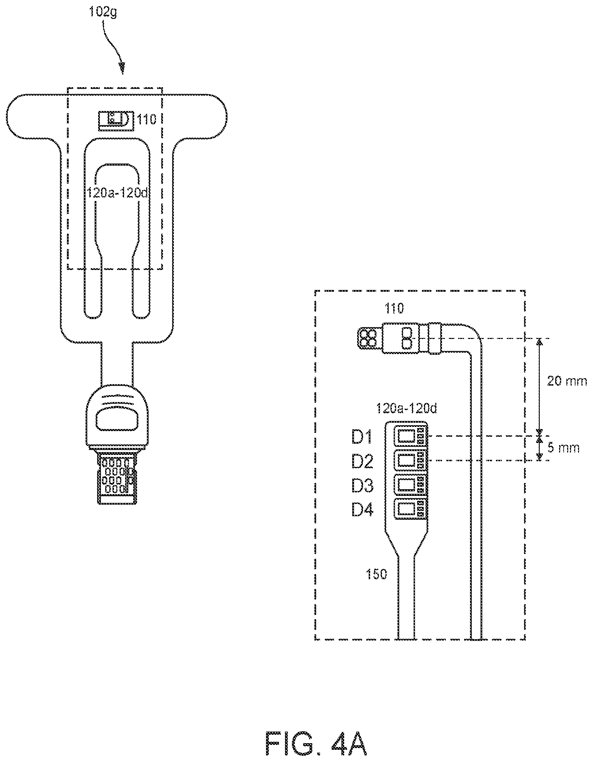

FIG. 4A illustrates acoustic emitter-detector separation distances for an embodiment of the sensor 102g illustrated in FIG. 1G. An embodiment of sensor 102g is shown on the left and a magnified image of the acoustic emitter-detector spacings in shown on the right. As shown on the right, in the illustrated embodiment, the acoustic emitter 110 is separated by the first detector by 20 mm. Each subsequent detector in the detector array 120a-d is an addition 5 mm away from the previous detector along the measurement axis of sensor 102g. Although FIG. 4A illustrates an embodiment with four acoustic detectors 120a-d, other embodiments may have number of two or more detectors. In addition, other embodiments may use different emitter-detector spacings (e.g., less than 30 mm, or less than 20 mm, or less than 15 mm, or less than 10 mm) and/or different detector-detector spacings (e.g., less than 10 mm, or less than 5 mm, or less than 3 mm), depending on the application.

In some embodiments, the blood pressure measurement systems 100 described herein use a series of sinusoidal signals at various different frequencies as the electrical input signals for measuring blood pressure. These sinusoidal signals are fed to the acoustic exciter 110, which then emits corresponding acoustic waves into the patient's body. Each of the input signals has a particular phase *φ*(*t*) = 2*πfτ*(*t*), where *f* is the frequency of the sinusoidal signal and τ is the elapsed time. In some embodiments, the processor of the blood pressure measurement systems 100 described herein uses the electrical output signals from the acoustic detectors 120a-d to determine the elapsed phase of the detected signals as compared to the input signals.

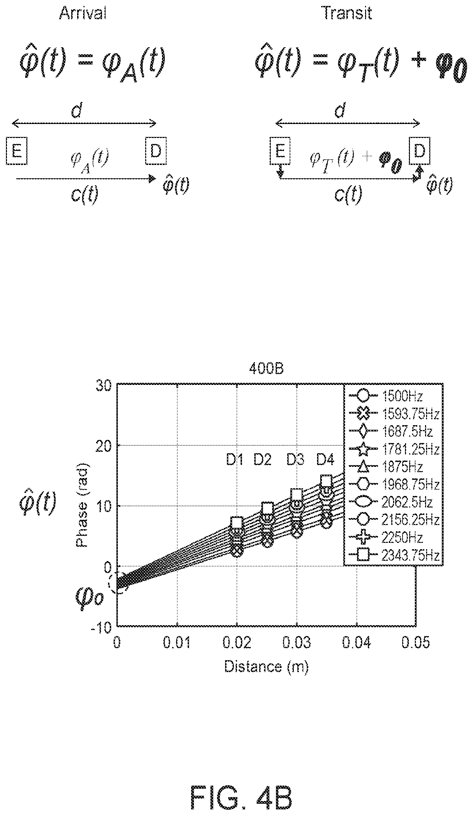

As summarized at the top of FIG. 4B, using the elapsed phase, the system 100 can calculate, for each desired moment in time, an arrival speed for each frequency component at each detector, where Arrival Speed = 2π*f*·(Distance / Elapsed Phase). Since blood pressure in the artery determines wave speed, the blood pressure can then be calculated. The system 100 can additionally or alternatively calculate, for each desired moment in time, a transit speed for each frequency component, where Transit Speed = 2π*f·*Distance / (Elapsed Phase - Delay Phase). The delay phase may be due to any number of causes, such as delay in exciter, overall system delay, etc. It can be subtracted from elapsed phase in order to obtain a transit time or speed that more closely approximates only the phase that elapsed during propagation through the artery, not elapsed phase that resulted from other miscellaneous delays. The detected arrival elapsed phase can be given as *φ̂*(*t*) = *φ_{T}*(*t*) + *φ*₀, the time-variant phase term is due to the transit of the wave through the artery, and the constant phase term encompasses other miscellaneous system delays.

FIG. 4B is a graph 400 of the elapsed phase detected by the sensor 102g in FIG. 4A for each of ten frequency components at each of the four acoustic detectors 120a-d for a particular moment in time. While ten frequency components are illustrated, any number can be used. The frequencies can be selected based on the acoustic emitter 110, the acoustic detectors 120, and the application. In graph 400, measured elapsed phase is plotted as a function of distance from the acoustic emitter 110. As shown in the graph, the first detector is located at 20 mm, the second detector is located at 25 mm, the third detector is located at 30 mm, and the fourth detector is located at 35 mm. Each dot corresponds to one of the transmitted frequency components. The elapsed phase is proportional to frequency, so higher frequencies have greater elapsed phases. Since there are 10 frequency components detected by four detectors, there are 40 graphed samples of elapsed phase. A line of best fit can be calculated for the four points corresponding to each frequency component. These lines of best fit are shown in graph 400. As illustrated, the lines of best fit for the respective frequency components tend to intercept the y-axis (zero distance from the acoustic emitter) at the value of the constant phase that is attributable to miscellaneous system delays rather than actual transit of the waves through the artery. Thus, this delay phase can be determined and subtracted out. In addition, the system can determine the slopes of the lines of best fit for the respective frequency components. These slopes (or their inverses) are related to wave speed. Using the above equations, the system 100 can calculate arrival speed and/or transit speed. As already discussed, the speed of propagation of the waves through the artery is dependent on the instantaneous blood pressure at the moment in question, so blood pressure measurements can thus be determined. This process can be repeated at the desired sampling rate to provide continuous blood pressure monitoring.

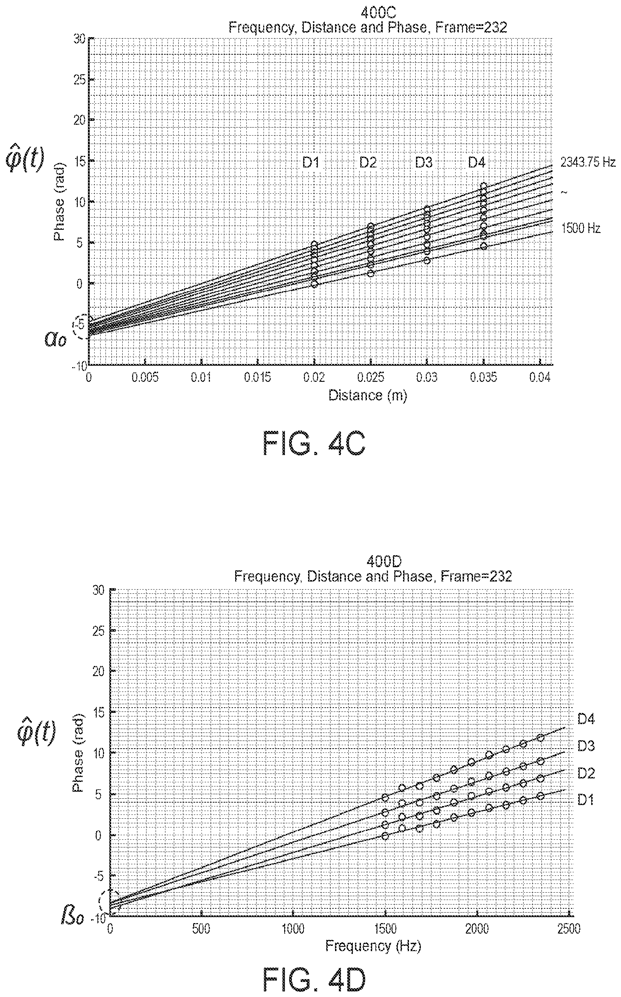

FIG. 4C is a graph 400c which further illustrates how the elapsed phase values measured by the acoustic detectors of the sensor in FIG. 4A for each frequency component can be used to determine slope values for lines of best fit so as to determine blood pressure values. Graph 400c once again plots elapsed phase as a function of distance for each of ten frequency components. The elapsed phase values for each frequency component can be used to calculate a line of best fit whose slope yields a blood pressure value according to the equation *φ̂*(*t*) = *α*₁*d* + *α*₀. This process can be repeated at the desired sampling rate to provide continuous blood pressure monitoring.

FIG. 4D is another graph 400d which further illustrates how the elapsed phase values measured by the acoustic detectors of the sensor in FIG. 4A for each frequency component can be used to determine slope values for lines of best fit so as to determine blood pressure values. In contrast to graph 400c in FIG. 4C, graph 400d plots elapsed phase as a function of frequency for each of the detectors. The ten elapsed phase values (corresponding to the ten frequency components) as measured by each detector can be used to calculate a line of best fit whose slope yields a blood pressure value according to the equation *φ̂*(*t*) = *β*₁*f* + *β*₀. This process can be repeated at the desired sampling rate to provide continuous blood pressure monitoring.

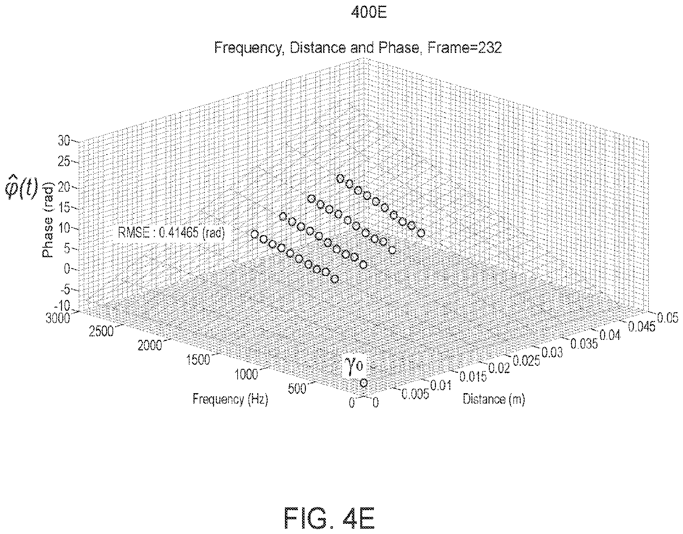

FIG. 4E is a graph 400e which illustrates how the elapsed phase values measured by the acoustic detectors of the sensor in FIG. 4A for each frequency component can be used to determine slope values for planes of best fit so as to determine blood pressure values. Graph 400e plots elapsed phase as both a function of distance and of frequency. The elapsed phase values for each frequency component can be used to calculate a plane of best fit whose slope yields a blood pressure value according to the equation *φ̂*(*t*) = *γ*₁*fd* + *γ*₀. This process can be repeated at the desired sampling rate to provide continuous blood pressure monitoring.

The foregoing techniques can be used to determine the speeds of the acoustic waves that propagate through the patient's artery. As already discussed, the speed of an acoustic wave propagating through the artery is dependent on the instantaneous blood pressure. A patient-specific calibration value can be used to convert wave speed to a blood pressure measurement for that patient. In some embodiments, the continuous blood pressure monitoring system 100 is used to take wave speed measurements while a second blood pressure measurement device takes one or more blood pressure measurements. The second blood pressure measurement device can be, for example, a cuff-based blood pressure measurement device or an arterial line. In some embodiments, the second blood pressure measurement device takes two blood pressure measurements, such as diastolic and systolic pressure, for a given time. These measurements can then be compared to the wave speed measurements captured by the continuous blood pressure monitoring system 100 at the given time. A calibration value that converts the wave speed measurements to the blood pressure measurements can then be determined. In some embodiments, the calibration process can be repeated at intervals to either verify the current calibration value for the patient or to determine a new calibration value. In some embodiments, the calibration interval is greater than 15 minutes, or greater than 1 hour, or greater than 2 hours, or greater than 3 hours, or greater than 4 hours, or greater than 5 hours, or greater than 10 hours, or greater than 1 day.

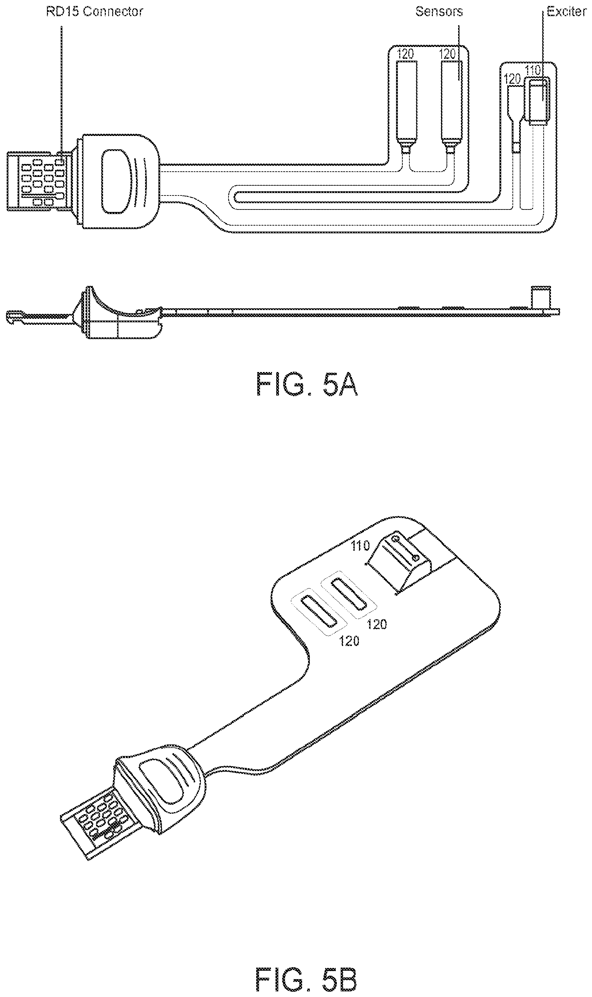

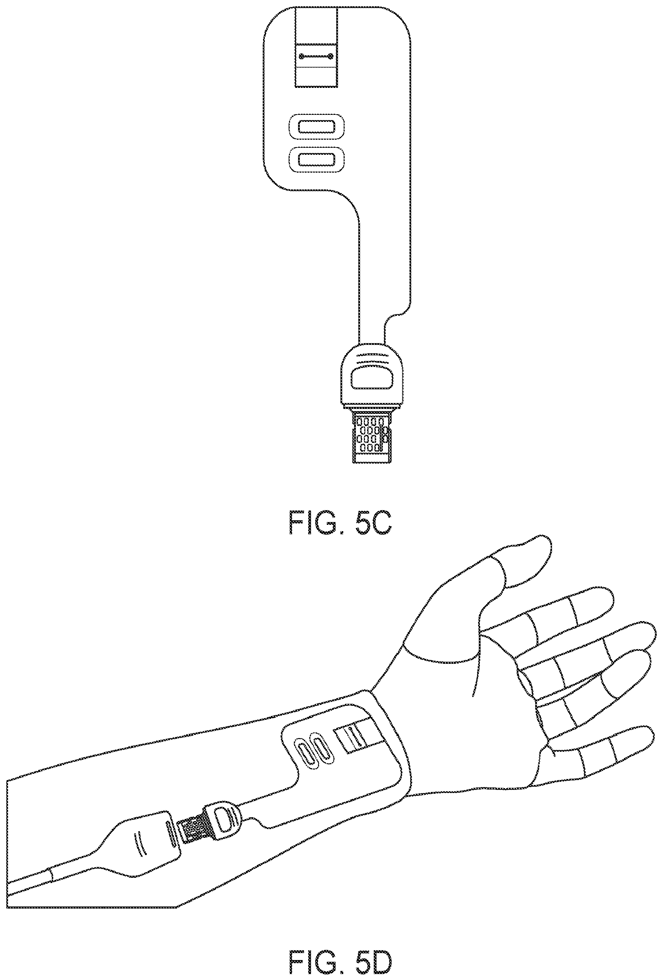

FIGS. 5A-5D illustrate example embodiments of sensors for the blood pressure monitoring system. FIG. 5A shows an example embodiment of a sensor with an acoustic exciter 110 and multiple acoustic detectors 120 provided on split substrate leads. FIG. 5B shows a perspective view of an example embodiment of a sensor with an acoustic exciter 110 and multiple acoustic detectors 120 provided on a common substrate. FIG. 5C shows a plan view of the sensor shown in FIG. 5B. FIG. 5D shows the sensor of FIG. 5B in the as-worn position on a patient's forearm.

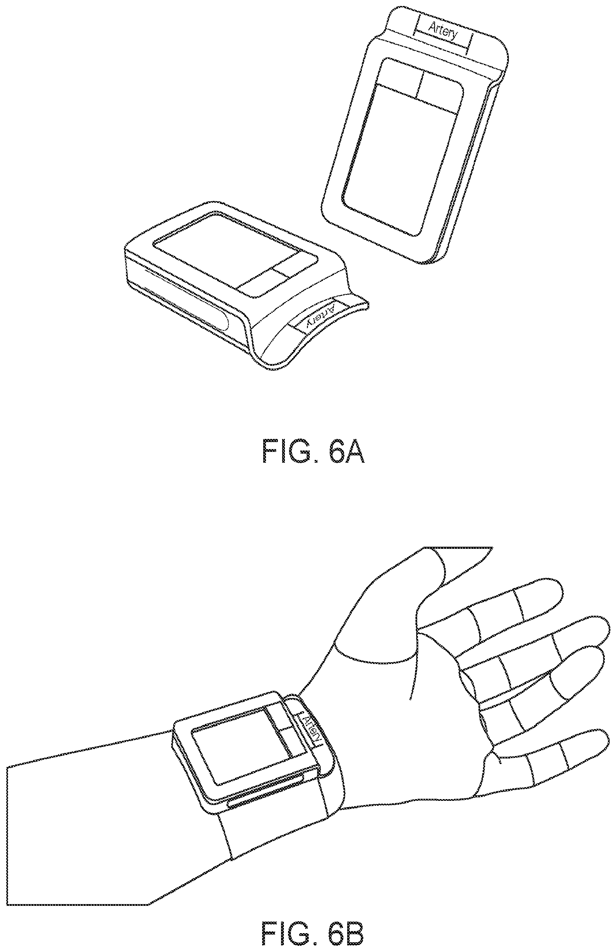

FIGS. 6A-6B illustrate an example embodiment of a blood pressure monitoring system with a built-in display. The illustrated embodiment can include all components of the blood pressure monitoring system (e.g., waveform/signal generator, processor, battery, etc.) in a single wearable package. The display can be used, for example, to output measurements to the user in graphical or text format. Though not seen, the acoustic exciter 110 and acoustic detector(s) can be provided in the underside of the unit so as to be in contact with the patient's skin when worn. As shown in FIG. 6A, the blood pressure monitoring system can also include an indicator to show how the unit should be positioned with respect to the patient's radial artery. FIG. 6B shows the blood pressure monitoring system attached to the patient's forearm with a wrist strap and worn like a wristwatch.

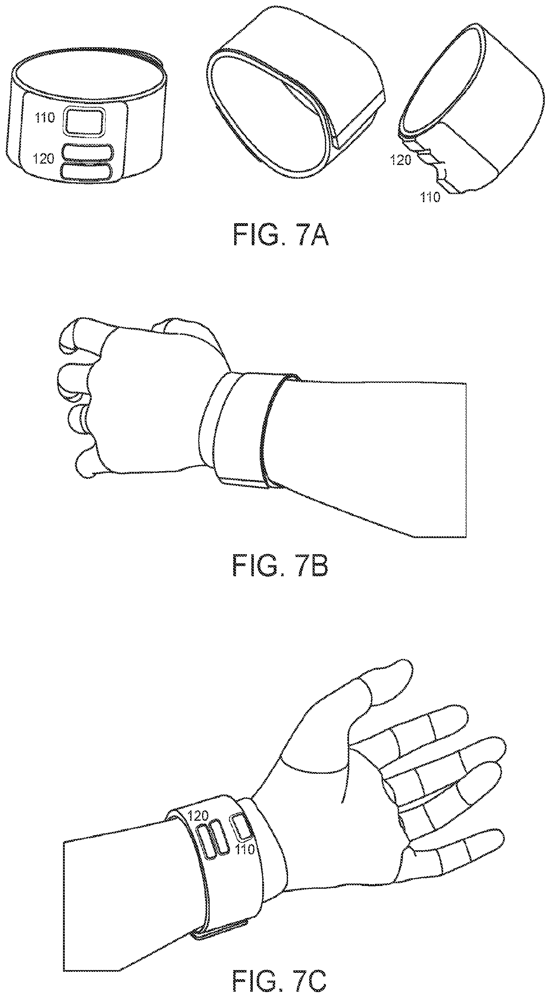

FIGS. 7A-7C illustrate an example embodiment of a blood pressure monitoring system with an acoustic exciter 110 and acoustic detectors 120 integrated in a wristband. The wristband can include a controller to provide a signal input to the acoustic exciter 110 and to capture output signals from the acoustic detectors 120. The wristband can also include a wireless communication module to provide data to an external device, such as a smartphone, to process the output signals and display the blood pressure measurements. The wristband can include windows through the wristband material to allow the acoustic exciter 110 and the acoustic detectors 120 to be in contact with the patient's skin when the wristband is worn.

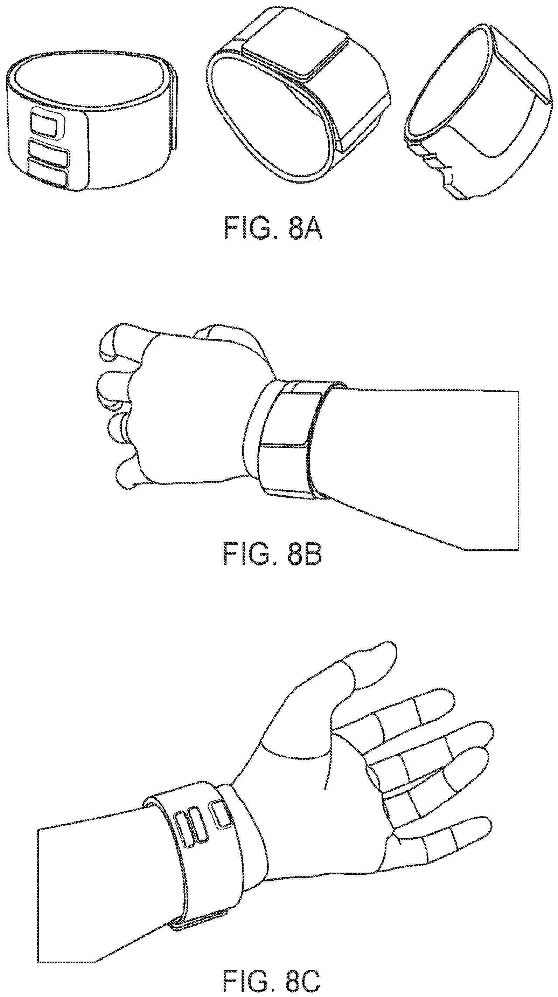

FIGS. 8A-8C illustrate an example embodiment of a blood pressure monitoring system which is similar to that of FIGS. 7A-7C but which additionally includes a built-in display.

## Claims

1. A blood pressure monitoring system comprising:
an acoustic exciter (110) configured to receive an electrical input signal and to produce an acoustic signal, the acoustic exciter being provided on a first substrate portion (114);
an acoustic detector array (120a-d) spaced apart from the acoustic exciter, the acoustic detector array being configured to detect the acoustic signal and to produce electrical output signals, the acoustic detector array being provided on a second substrate portion (124) that is acoustically decoupled from the first substrate portion;
an acoustic detector (120e) located directly adjacent to the acoustic exciter and configured to measure the acoustic signal produced by the acoustic exciter; and
a processor configured to determine a blood pressure measurement from the electrical output signals.

2. The blood pressure monitoring system of Claim 1, wherein the first and second substrate portions are separated by a gap.

3. The blood pressure monitoring system of Claim 1, wherein the first and second substrate portions are separated by acoustically absorptive material.

4. The blood pressure monitoring system of Claim 1, wherein the processor is configured to determine the blood pressure measurement based on a measured phase delay between the electrical input signal and the electrical output signals.

5. The blood pressure monitoring system of Claim 1, wherein the first and second substrate portions are flexible.

6. The blood pressure monitoring system of Claim 1, further comprising a flexible circuit that connects the acoustic exciter and the acoustic detector array to an electrical connector.

7. The blood pressure monitoring system of Claim 1, wherein the acoustic exciter and the acoustic detector array comprise a piezo device or a microelectromechanical system.

8. The blood pressure monitoring system of Claim 1, further comprising an attachment element configured to attach the acoustic exciter and the acoustic detector array to the forearm of a patient over the radial artery.

9. The blood pressure monitoring system of Claim 8, further comprising an alignment indicator to align a measurement axis of the acoustic exciter and the acoustic detector array to the radial artery.

10. The blood pressure monitoring system of Claim 2, wherein the gap exists along a straight line path from the acoustic exciter to the acoustic detector array.

11. The blood pressure monitoring system of Claim 1, further comprising a first bypass substrate portion connecting the first substrate portion and the second substrate portion, wherein a bypass acoustic signal path from the acoustic exciter to the acoustic detector array via the first bypass substrate portion is longer than the straight line path from the acoustic exciter to the acoustic detector array.

12. The blood pressure monitoring system of Claim 11, wherein the bypass acoustic signal path is at least two times longer than the straight line distance between the acoustic exciter and the acoustic detector array.

13. The blood pressure monitoring system of Claim 11, wherein the bypass acoustic signal path is at least five times longer than the straight line distance between the acoustic exciter and the acoustic detector array.

14. The blood pressure monitoring system of Claim 11, wherein the bypass acoustic signal path comprises acoustically absorptive material.

15. The blood pressure monitoring system of Claim 1, wherein the processor is further configured to:
determine one or more characteristics of the acoustic signal based on an electrical output signal from the acoustic detector located directly adjacent to the acoustic exciter; and
adjust the electrical output signals from the acoustic detector array and/or the blood pressure measurement based on the one or more characteristics of the acoustic signal.

## Patentansprüche

1. Blutdrucküberwachungssystem, das Folgendes umfasst:
einen akustischen Erreger (110), der konfiguriert ist, ein elektrisches Eingangssignal zu empfangen und ein akustisches Signal zu erzeugen, wobei der akustische Erreger auf einem ersten Substratabschnitt (114) bereitgestellt ist;
eine Anordnung (120a-d) akustischer Detektoren, die von dem akustischen Erreger getrennt ist, wobei die Anordnung akustischer Detektoren konfiguriert ist, das akustische Signal zu detektieren und elektrische Ausgangssignale zu erzeugen, wobei die Anordnung akustischer Detektoren auf einem zweiten Substratabschnitt (124) bereitgestellt ist, der von dem ersten Substratabschnitt akustisch entkoppelt ist;
einen akustischen Detektor (120e), der sich direkt neben dem akustischen Erreger befindet und konfiguriert ist, das durch den akustischen Erreger erzeugte akustische Signal zu messen; und
einen Prozessor, der konfiguriert ist, eine Blutdruckmessung aus den elektrischen Ausgangssignalen zu bestimmen.

2. Blutdrucküberwachungssystem nach Anspruch 1, wobei der erste und der zweite Substratabschnitt durch einen Spalt getrennt sind.

3. Blutdrucküberwachungssystem nach Anspruch 1, wobei der erste und der zweite Substratabschnitt durch akustisch absorbierendes Material getrennt sind.

4. Blutdrucküberwachungssystem nach Anspruch 1, wobei der Prozessor konfiguriert ist, die Blutdruckmessung basierend auf einer gemessenen Phasenverzögerung zwischen dem elektrischen Eingangssignal und den elektrischen Ausgangssignalen zu bestimmen.

5. Blutdrucküberwachungssystem nach Anspruch 1, wobei der erste und der zweite Substratabschnitt flexibel sind.

6. Blutdrucküberwachungssystem nach Anspruch 1, das ferner eine flexible Schaltung umfasst, die den akustischen Erreger und die Anordnung akustischer Detektoren mit einem elektrischen Verbinder verbindet.

7. Blutdrucküberwachungssystem nach Anspruch 1, wobei der akustische Erreger und die Anordnung akustischer Detektoren eine Piezovorrichtung oder ein mikroelektromechanisches System umfassen.

8. Blutdrucküberwachungssystem nach Anspruch 1, das ferner ein Befestigungselement umfasst, das konfiguriert ist, den akustischen Erreger und die Anordnung akustischer Detektoren über der Speichenarterie am Unterarm eines Patienten zu befestigen.

9. Blutdrucküberwachungssystem nach Anspruch 8, das ferner einen Ausrichtungsindikator zum Ausrichten einer Messachse des akustischen Erregers und der Anordnung akustischer Detektoren auf die Speichenarterie umfasst.

10. Blutdrucküberwachungssystem nach Anspruch 2, wobei der Spalt entlang eines geradlinigen Pfades von dem akustischen Erreger zu der Anordnung akustischer Detektoren vorhanden ist.

11. Blutdrucküberwachungssystem nach Anspruch 1, das ferner einen ersten Umgehungs-Substratabschnitt umfasst, der den ersten Substratabschnitt und den zweiten Substratabschnitt verbindet, wobei ein akustischer Umgehungs-Signalpfad von dem akustischen Erreger über den ersten Umgehungs-Substratabschnitt zu der Anordnung akustischer Detektoren länger als der geradlinige Pfad von dem akustischen Erreger zu der Anordnung akustischer Detektoren ist.

12. Blutdrucküberwachungssystem nach Anspruch 11, wobei der akustische Umgehungs-Signalpfad wenigstens zweimal länger als der geradlinige Abstand zwischen dem akustischen Erreger und der Anordnung akustischer Detektoren ist.

13. Blutdrucküberwachungssystem nach Anspruch 11, wobei der akustische Umgehungs-Signalpfad wenigstens fünfmal länger als der geradlinige Abstand zwischen dem akustischen Erreger und der Anordnung akustischer Detektoren ist.

14. Blutdrucküberwachungssystem nach Anspruch 11, wobei der akustische Umgehungs-Signalpfad akustisch absorbierendes Material umfasst.

15. Blutdrucküberwachungssystem nach Anspruch 1, wobei der Prozessor ferner konfiguriert ist:
eine oder mehrere Eigenschaften des akustischen Signals basierend auf einem elektrischen Ausgangssignal von dem akustischen Detektor, der sich direkt neben dem akustischen Erreger befindet, zu bestimmen; und
die elektrischen Ausgangssignale von der Anordnung akustischer Detektoren und/oder die Blutdruckmessung basierend auf der einen oder den mehreren Eigenschaften des akustischen Signals einzustellen.

## Revendications

1. Système de surveillance de la pression artérielle comprenant :
un excitateur acoustique (110) configuré pour recevoir un signal électrique d'entrée et pour produire un signal acoustique, l'excitateur acoustique étant disposé sur une première portion de substrat (114) ;
un réseau de détecteurs acoustiques (120a-d) espacé de l'excitateur acoustique, le réseau de détecteurs acoustiques étant configuré pour détecter le signal acoustique et pour produire des signaux électriques de sortie, le réseau de détecteurs acoustiques étant disposé sur une deuxième portion de substrat (124) acoustiquement découplée de la première portion de substrat ;
un détecteur acoustique (120e) situé immédiatement adjacent à l'excitateur acoustique et configuré pour mesurer le signal acoustique produit par l'excitateur acoustique ; et
un processeur configuré pour déterminer une mesure de pression artérielle à partir des signaux électriques de sortie.

2. Système de surveillance de la pression artérielle selon la revendication 1, dans lequel les première et deuxième portions de substrat sont séparées par un espace.

3. Système de surveillance de la pression artérielle selon la revendication 1, dans lequel les première et deuxième portions de substrat sont séparées par un matériau acoustiquement absorbant.

4. Système de surveillance de la pression artérielle selon la revendication 1, dans lequel le processeur est configuré pour déterminer la mesure de pression artérielle sur la base d'un retard de phase mesuré entre le signal électrique d'entrée et les signaux électriques de sortie.

5. Système de surveillance de la pression artérielle selon la revendication 1, dans lequel les première et deuxième portions de substrat sont flexibles.

6. Système de surveillance de la pression artérielle selon la revendication 1, comprenant en outre un circuit flexible reliant l'excitateur acoustique et le réseau de détecteurs acoustiques à un connecteur électrique.

7. Système de surveillance de la pression artérielle selon la revendication 1, dans lequel l'excitateur acoustique et le réseau de détecteurs acoustiques comprennent un dispositif piézoélectrique ou un système microélectromécanique.

8. Système de surveillance de la pression artérielle selon la revendication 1, comprenant en outre un élément de fixation configuré pour fixer l'excitateur acoustique et le réseau de détecteurs acoustiques à l'avant-bras d'un patient au-dessus de l'artère radiale.

9. Système de surveillance de la pression artérielle selon la revendication 8, comprenant en outre un indicateur d'alignement pour aligner un axe de mesure de l'excitateur acoustique et du réseau de détecteurs acoustiques avec l'artère radiale.

10. Système de surveillance de la pression artérielle selon la revendication 2, dans lequel l'espace existe le long d'un trajet en ligne droite depuis l'excitateur acoustique jusqu'au réseau de détecteurs acoustiques.

11. Système de surveillance de la pression artérielle selon la revendication 1, comprenant en outre une première portion de substrat de dérivation reliant la première portion de substrat et la deuxième portion de substrat, dans lequel un trajet de signal acoustique de dérivation depuis l'excitateur acoustique jusqu'au réseau de détecteurs acoustiques par l'intermédiaire de la première portion de substrat de dérivation est plus long que le trajet en ligne droite depuis l'excitateur acoustique jusqu'au réseau de détecteurs acoustiques.

12. Système de surveillance de la pression artérielle selon la revendication 11, dans lequel le trajet de signal acoustique de dérivation est au moins deux fois plus long que la distance en ligne droite entre l'excitateur acoustique et le réseau de détecteurs acoustiques.

13. Système de surveillance de la pression artérielle selon la revendication 11, dans lequel le trajet de signal acoustique de dérivation est au moins cinq fois plus long que la distance en ligne droite entre l'excitateur acoustique et le réseau de détecteurs acoustiques.

14. Système de surveillance de la pression artérielle selon la revendication 11, dans lequel le trajet de signal acoustique de dérivation comprend un matériau acoustiquement absorbant.

15. Système de surveillance de la pression artérielle selon la revendication 1, dans lequel le processeur est en outre configuré pour :
déterminer une ou plusieurs caractéristiques du signal acoustique sur la base d'un signal électrique de sortie provenant du détecteur acoustique situé immédiatement adjacent à l'excitateur acoustique ; et
ajuster les signaux électriques de sortie provenant du réseau de détecteurs acoustiques et/ou la mesure de pression artérielle sur la base de la ou des caractéristiques du signal acoustique.
